# EUROPÄISCHE PATENTSCHRIFT

(11) **EP 2 151 185 B1**
(45) Veröffentlichungstag und Bekanntmachung des Hinweises auf die Patenterteilung: **22.02.2012**
(21) Anmeldenummer: 09166358.3
(22) Anmeldetag: 24.07.2009
(51) Int. Cl.: A61B 1/267

(54) **Laryngoskopspatel**
Laryngoscope applicator
Spatule de laryngoscope

(30) Priorität: 05.08.2008 DE 102008036826
(43) Veröffentlichungstag der Anmeldung: 10.02.2010
(73) Patentinhaber: Karl Storz GmbH & Co. KG, 78532 Tuttlingen (DE)
(72) Erfinder: Fridolin, Anders, 78194, Immendingen-Zimmern (DE)
(74) Vertreter: Witte, Weller & Partner

(56) Entgegenhaltungen:
- DE-A1- 1 937 817
- DE-A1- 2 621 232
- DE-A1- 3 119 725
- DE-A1- 3 317 831
- DE-B1- 1 766 713
- DE-U1- 20 004 324

## Beschreibung

Die Erfindung betrifft einen Laryngoskopspatel, mit einem Spatelblatt, wobei das Spatelblatt im Querschnitt zwei Seitenwände, die zumindest über eine Teillänge des Spatelblattes voneinander beabstandet sind, und zwei Schenkel aufweist, von denen ein erster Schenkel sich von der ersten Seitenwand aus über die zweite Seitenwand hinaus erstreckt und ein zweiter Schenkel sich von der zweiten Seitenwand aus über die erste Seitenwand hinaus erstreckt, wobei die Seitenwände und die Schenkel einen Hohlraum ausbilden.

Die Erfindung betrifft ferner ein Verfahren zur Herstellung eines Spatelblattes eines Laryngoskopspatels.

Ein Laryngoskopspatel der eingangs genannten Art ist aus den Dokumenten DE 31 19 725 C2 und DE 33 17 831 C2 jeweils bekannt.

Ein solcher Laryngoskopspatel wird an einem Griff angeordnet und als Laryngoskop in der Laryngoskopie und vor allem bei der endotrachealen Intubation verwendet. Speziell zum letzteren Zweck weist der Laryngoskopspatel im Querschnitt ein Profil mit einem abgestuften Aufbau auf, der z.B. eine Führung für einen bei einer Intubation verwendeten Endotrachealtubus bietet. Ferner ist im distalen Bereich des Laryngoskopspatels eine Lichtquelle oder ein Lichtaustrittsende eines Lichtleiters angeordnet, um dem Operateur eine Betrachtung des Kehlkopfs zu ermöglichen.

Waren diese Lichtquellen früher direkt an dem betreffenden Ende angeordnete direkte Lichtquellen, wie z.B. Glühlampen, so werden heute vorzugsweise Lichtleiter, wie z.B. Glasfaser, verwendet, die ein durch eine im Griffbereich befindliche Lichtquelle erzeugtes Licht durch den Laryngoskopspatel zur betreffenden distalen Stelle leiten, wo das Licht dann austreten kann.

Für die Anordnung des Lichtleiters am Laryngoskopspatel weist dieser den oben erwähnten Hohlraum auf, in dem der Lichtleiter geschützt angeordnet ist.

Der vorstehend genannte Lichtleiter kann jedoch bei einem erfindungsgemäßen Laryngoskopspatel nicht nur dazu dienen, Licht von proximal nach distal zuzuführen, um Licht in den Rachenraum abzustrahlen, sondern auch umgekehrt dazu, Licht aus dem Rachenraum zur Bildaufnahme aufzunehmen, d.h. Licht kann in den Lichtleiter am distalen Ende des Lichtleiters eintreten und nach proximal geleitet werden.

Des Weiteren kann bei einem erfindungsgemäßen Laryngoskopspatel der Hohlraum dazu dienen, elektrische Leitungen geschützt aufzunehmen, die beispielsweise dazu dienen, eine im distalen Bereich des Laryngoskopspatels angeordnete Lichtquelle, beispielsweise eine LED, mit Spannung zu versorgen, und/oder es kann im distalen Bereich des Laryngoskopspatels ein elektronischer Bildaufnehmer angeordnet sein, wobei dann in dem Hohlraum elektrische Leitungen verlaufen, über die der elektronische Bildaufnehmer mit Spannung versorgt wird bzw. über die Signale vom elektronischen Bildaufnehmer nach proximal in den Handgriff geführt werden.

Der eingangs genannte Aufbau des Spatelblattes wird bei dem aus dem Dokument DE 31 19 725 C2 bekannten Laryngoskopspatel dadurch erreicht, dass zwei im Querschnitt L-förmige Längsprofile unter Bildung des Hohlraums aneinander angesetzt werden. Die beiden L-förmigen Längsprofile sind dabei im Querschnitt um 180° versetzt zueinander angeordnet. Dabei stößt ein Ende eines Schenkels des ersten L-Profils an einen Schenkel des zweiten L-Profils an, und das Ende des anderen Schenkels des zweiten L-Profils stößt an den anderen Schenkel des ersten L-Profils an. An diesen Stoßkanten sind die beiden L-Profile miteinander verlötet.

Der aus dem Dokument DE 33 17 831 C2 bekannte Laryngoskopspatel weist ein Spatelblatt auf, das aus einem im Querschnitt Z-förmigen Profilteil und einem im Querschnitt L-förmigen Profilteil zusammengesetzt ist, um den Hohlraum zu bilden. Dabei wird das L-förmige Profilteil mit den freien Enden seiner beiden Schenkel entlang von Stoßkanten an das Z-förmige Profilteil angesetzt und entlang der Stoßkanten mit dem Z-förmigen Profilteil verschweißt. Die Schweißnähte werden anschließend durch Feinschleifen und Feinpolieren geglättet.

Damit ein Laryngoskopspatel, wie er zuvor beschrieben wurde, mehrmals verwendet werden kann, ist es notwendig, dass dieser gut gereinigt und sterilisiert werden kann, weswegen er keine Vertiefungen, Ecken oder Kanten aufweisen sollte, in denen sich Bakterien oder anderes Material festsetzen kann. Ferner sollte auch ein Eindringen von Flüssigkeiten und/oder Bakterien in den Innenraum des Spatelblattes vermieden werden, da auch hier ein ungehindertes Bakterienwachstum möglich wäre.

Zu diesem Zweck ist es bei den zuvor beschriebenen bekannten Laryngoskopspateln nötig, dass die beiden Profilteile dicht miteinander verschweißt bzw. verlötet werden und weiterhin diese Löt- bzw. Schweißstellen nachträglich aufwändig bearbeitet werden, so dass eine gleichmäßige Oberfläche erzeugt wird. Daran schließt sich meist noch ein Galvanisierungsschritt an, damit eine in optimaler Weise glatte Oberfläche erzeugt wird und jegliche Materialungleichmäßigkeiten, die das spätere Reinigen erschweren würden, beseitigt werden. Die bekannten Laryngoskopspatel sind somit in ihrer Gestaltung kostenaufwändig, und die Herstellung der bekannten Laryngoskopspatel ist darüber hinaus zu aufwändig.

Es ist daher eine Aufgabe der vorliegenden Erfindung, einen Laryngoskopspatel zu entwickeln, der einfach zu reinigen ist und dessen Herstellung dennoch möglichst weniger Nachbearbeitungsschritte bedarf und somit relativ einfach ist.

Ferner ist es eine Aufgabe der vorliegenden Erfindung, ein Verfahren zur Herstellung eines Spatelblattes eines solchen Laryngoskopspatels bereitzustellen.

Erfindungsgemäß wird die Aufgabe hinsichtlich des eingangs genannten Laryngoskopspatels dadurch gelöst, dass das Spatelblatt aus einer Ober- und einer Unterschale gebildet ist, die jeweils im Querschnitt einen ersten Abschnitt und zwei zueinander entgegengesetzt verlaufende zweite und dritte Abschnitte aufweisen, wobei die zweiten Abschnitte der Ober- und Unterschale flächig aufeinanderliegend den ersten Schenkel und die dritten Abschnitte flächig aufeinanderliegend den zweiten Schenkel bilden, während die ersten Abschnitte die Seitenwände bilden.

Die Aufgabe wird hinsichtlich des Verfahrens zum Herstellen eines Spatelblattes eines Laryngoskopspatels erfindungsgemäß dadurch gelöst, dass es folgende Schritte aufweist:
- Bereitstellen einer Ober- und einer Unterschale, die jeweils im Querschnitt einen ersten Abschnitt und zwei zueinander entgegengesetzt verlaufende zweite und dritte Abschnitte aufweisen,
- flächiges Aufeinanderlegen der zweiten Abschnitte und der dritten Abschnitte von Ober- und Unterschale,
- festes Verbinden der Ober- und Unterschale miteinander durch Fügen der zweiten und dritten Abschnitte der Ober- und Unterschale, so dass die zweiten Abschnitte einen ersten Schenkel und die dritten Abschnitte einen zweiten Schenkel bilden und die ersten Abschnitte Seitenwände bilden, wodurch zumindest über eine Teillänge des Spatelblattes ein Hohlraum ausgebildet wird.

Der erfindungsgemäße Laryngoskopspatel weist somit ein Spatelblatt auf, das durch Zusammenfügen einer Oberschale und einer Unterschale gebildet ist, die beide ein im Querschnitt im Wesentlichen Z-förmiges Profil aufweisen. Die im Wesentlichen Z-förmige Oberschale und die im Wesentlichen Z-förmige Unterschale werden mit gleicher Orientierung des "Z" seitlich zueinander versetzt angeordnet, so dass die zweiten und dritten Abschnitte der Ober- und Unterschale flächig aufeinander liegen, während die ersten Abschnitte, d.h. die Mittelschenkel der Z-Profile, die voneinander beabstandeten Seitenwände des Spatelblattes bilden. Durch das flächige Aufeinanderliegen der zweiten und dritten Abschnitte der Ober- und Unterschale werden Stoßkanten, wie sie im Stand der Technik vorhanden sind, vermieden, die schwierig durch Löten oder Schweißen zu fügen sind und insbesondere eine aufwändige Nachbearbeitung erfordern, um eine glatte Oberfläche zu erzielen. Bei dem erfindungsgemäßen Laryngoskopspatel bilden die Ober- und die Unterschale jeweils die vollständige Oberfläche des Spatelblatts aus. Dies bewirkt, dass die Oberfläche des Spatelblattes auf seiner oberen und unteren Seite frei von Schweißnähten ist, die einer Nachbearbeitung bedürfen. Der erfindungsgemäße Laryngoskopspatel ist daher gegenüber den bekannten Laryngoskopspateln mit weniger Arbeitsschritten herstellbar, was auch die Gestehungskosten des erfindungsgemäßen Laryngoskopspatels vermindert.

Das erfindungsgemäße Herstellungsverfahren ist einfach durchzuführen und erfordert wegen des flächigen Aufeinanderliegens der Ober- und Unterschale über die zweiten und dritten Abschnitte keine aufwändige Nachbearbeitung von Schweißnähten entlang von Stoßkanten.

In einer bevorzugten Ausgestaltung der Erfindung ist der Hohlraum am distalen Ende durch eine sich quer zur Längsrichtung des Spatelblattes erstreckende Stirnwand abgeschlossen, und die Stirnwand ist einstückig mit dem ersten Abschnitt und dem zweiten Abschnitt der Oberschale ausgebildet.

Die Stirnwand bewirkt, dass der Hohlraum distalseitig verschlossen ist und somit auch in diesen keine Flüssigkeiten etc. von distaler Seite her eintreten können.

Durch das einstückige Ausbilden mit dem ersten und zweiten Abschnitt der Oberschale entfällt ein nachträgliches Einsetzen einer solchen Stirnwand, wodurch auch kein nachträgliches Bearbeiten der dadurch entstehenden Verbindungsstellen erforderlich ist, weil von vornherein eine durchgehende Oberfläche ohne Unebenheiten erzeugt wird.

In der Stirnwand ist vorzugsweise zumindest eine Öffnung zur vorzugsweise dichten Aufnahme eines distalen Lichtaustrittsendes und/oder Lichteintrittsendes eines Lichtleiters zur Beleuchtung und/oder Bildaufnahme, zur Aufnahme einer Lichtquelle, beispielsweise einer LED, und/oder zur Aufnahme eines elektronischen Bildaufnehmers vorhanden.

In weiteren bevorzugten Ausgestaltungen der Erfindung ist die Unterschale durch ein Umformverfahren, vorzugsweise durch Hydroforming, hergestellt, wobei der erste Abschnitt mit dem zweiten und dritten Abschnitt einstückig geformt ist, und/oder die Oberschale ist durch ein Umformverfahren, vorzugsweise durch Hydroforming, hergestellt, wobei der erste Abschnitt mit dem zweiten und dritten Abschnitt und der Stirnwand einstückig geformt ist.

Diese Maßnahmen haben den Vorteil, dass Ober- sowie Unterschale aus einem Werkstück hergestellt werden können, weswegen sie jeweils für sich eine durchgehende geschlossene Oberfläche aufweisen. Man erhält somit durchgehende glatte Oberflächen, die gut zu reinigen sind. Hydroforming ist auch besonders geeignet, die gewünschte dreidimensionale geometrische Form der Ober- und Unterschale herzustellen.

Außerdem wird die Herstellung vereinfacht, da Ober- und/oder Unterschale jeweils aus nur einem Teil gefertigt werden, was Zeit und Kosten spart.

In einer weiteren bevorzugten Ausgestaltung der Erfindung sind die Ober- und die Unterschale über die aufeinanderliegenden zweiten und dritten Abschnitte miteinander verklebt.

Das Verkleben der aufeinanderliegenden Flächen der zweiten und dritten Abschnitte der Ober- und Unterschale hat den Vorteil, dass es recht einfach, schnell und kostengünstig durchführbar ist. Außerdem bietet diese Fügetechnik aufgrund der flächigen Verbindung einen sicheren Halt der Ober- und Unterschale aneinander. Darüber hinaus ist die Außenseite des Spatelblatts in diesem Fall völlig frei von Fügestellen.

In einer weiteren bevorzugten Ausgestaltung der Erfindung sind die Ober- und die Unterschale miteinander an einer Kontaktkante, die jeweils aneinandergrenzende Kanten der zweiten und dritten Abschnitte miteinander bilden, umlaufend verlötet.

Durch das Verlöten an der Kontaktkante wird nur am äußeren umlaufenden Bereich eine Verbindung hergestellt, die relativ einfach und schnell nachzubearbeiten ist. Damit gibt es nur eine Verbindungsnaht zwischen Ober- und Unterschale, was die Möglichkeiten für das Festsetzen von Bakterien und das Eindringen von Flüssigkeiten auf ein Minimum reduziert. Außerdem wird das Herstellungsverfahren mit nur einem Herstellungsschritt für die Verbindung der Schalen deutlich verkürzt.

In einer weiteren bevorzugten Ausgestaltung der Erfindung sind die Ober- und die Unterschale miteinander an einer Kontaktkante, die jeweils aneinandergrenzende Kanten der zweiten und dritten Abschnitte miteinander bilden, vorzugsweise durch Laserschweißen, umlaufend verschweißt.

Auch hier ist der verschweißte Bereich ggf. auf einfache Weise nachzubearbeiten, was aber speziell beim Laserschweißen nicht nötig ist, da Laserschweißen eine sehr glatte Verbindungsstelle ergibt, die keiner weiteren Nachbehandlung bedarf. Dieses Verfahren ist somit zeit- und kostensparend.

In einer weiteren bevorzugten Ausgestaltung der Erfindung weist das Spatelblatt am distalen Ende eine abgerundete Spatelspitze auf, wobei die Spatelspitze einstückig mit der Oberschale und/oder der Unterschale geformt ist.

Eine abgerundete Spatelspitze hat den Vorteil, dass das Risiko einer Verletzung beim Einführen des Laryngoskopspatels durch den Mundraum Richtung Kehlkopf auf ein Minimum reduziert wird. Die einstückige Bauweise der Spatelspitze mit der Ober- und/oder Unterschale hat den Vorteil, dass die Spatelspitze nicht nachträglich an das Spatelblatt angebracht werden muss und somit ein weiterer Schritt bei der Herstellung eingespart wird, was das Herstellungsverfahren noch günstiger und schneller macht.

Alternativ zur vorstehend genannten einstückigen Bauweise kann die Spatelspitze, wenn dies zur vereinfachten Herstellung der Unter- und/oder Oberschale beiträgt, auf das Spatelblatt aufgeschweißt, aufgelötet oder aufgeklebt werden.

Weitere Vorteile und Merkmale ergeben sich aus der nachfolgenden Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachfolgend anhand eines ausgewählten Ausführungsbeispiels im Zusammenhang mit den beiliegenden Zeichnungen näher beschrieben und erläutert. Es zeigen:
- Fig. 1: eine Seitenansicht eines Laryngoskops;
- Fig. 2: einen Schnitt längs der Linie II-II in Fig. 1;
- Fig. 3: eine perspektivische Ansicht einer Unterschale, die Teil eines Spatelblatts des Laryngoskops in Fig. 1 ist;
- Fig. 4: eine perspektivische Seitenansicht einer Oberschale, die ebenfalls Teil des Spatelblatts des Laryngoskops in Fig. 1 ist.

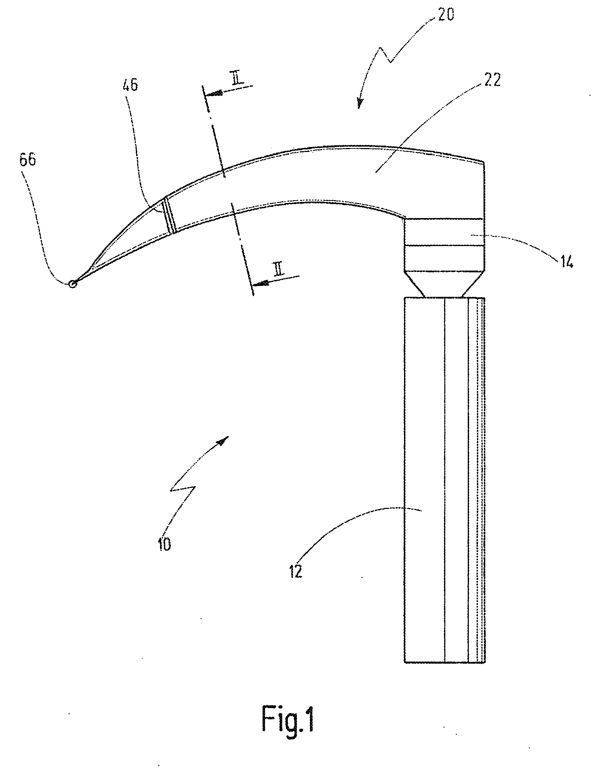

In Fig. 1 ist ein mit dem allgemeinen Bezugszeichen 10 versehenes Laryngoskop in seiner Gesamtheit dargestellt.

Das Laryngoskop 10 ist aus einem Griff 12, einem Kopf 14 und einem Laryngoskopspatel 20 aufgebaut.

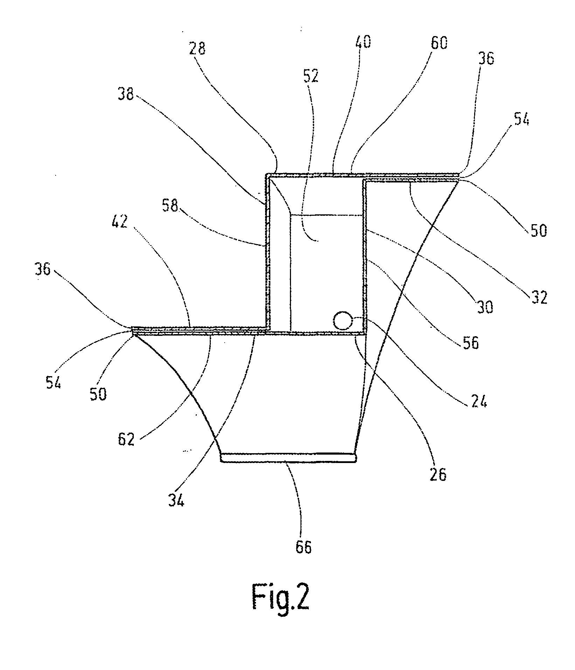

Einzelheiten des Laryngoskopspatels 20 sind in Fig. 2 bis 4 gezeigt.

Der Laryngoskopspatel 20 weist ein Spatelblatt 22 und einen darin angeordneten Lichtleiter 24 (Fig. 2) auf. Dieser Laryngoskopspatel 20 ist mit einem hier schematisch gezeigten Kopf 14 verbunden, der dann seinerseits mit dem Griff 12 verbunden ist.

Anstelle oder zusätzlich zu dem Lichtleiter 24 können in dem Spatelblatt 22 eine oder mehrere elektrische Leitungen verlaufen, die zur Spannungsversorgung für eine oder mehrere Lichtquellen, beispielsweise LEDs, im distalen Bereich des Spatelblatts 22 oder als Signalleitung(en) für einen im distalen Bereich angeordneten elektronischen Bildaufnehmer dienen.

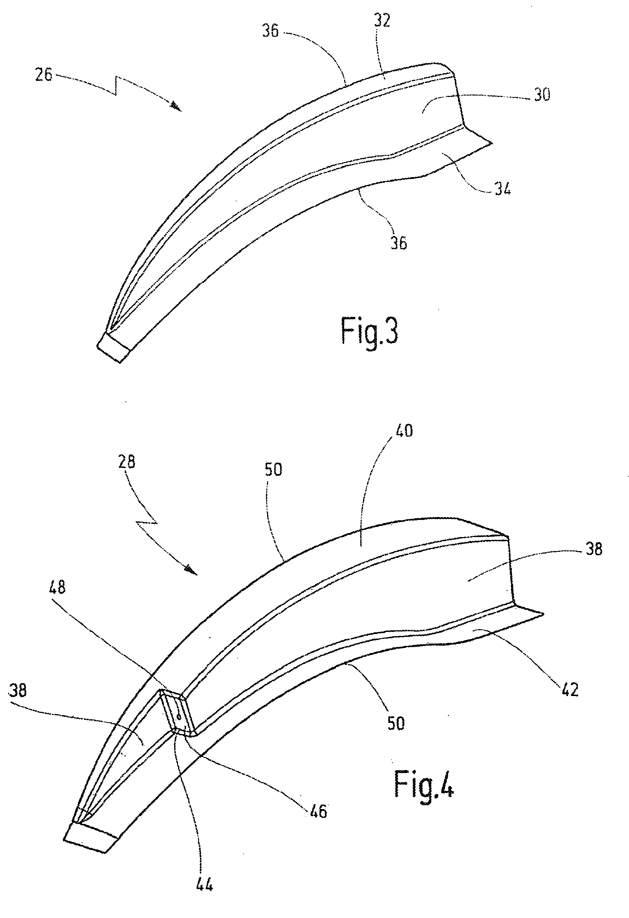

Das hier gezeigte Spatelblatt 22 weist eine an die anatomischen Gegebenheiten des Mund-/Rachenraums eines Patienten angepasste gebogene Form auf und ist aus einer Unterschale 26 und einer Oberschale 28 aufgebaut (Fig. 3 und Fig. 4).

Die Unterschale 26 ist aus einem ersten Abschnitt 30 und einem zweiten und dritten Abschnitt 32 und 34 aufgebaut, die sich von jeweils einem Ende des ersten Abschnitts 30 quer zu diesem in entgegengesetzte Richtungen weg erstrecken. Die Abschnitte 32 und 34 stehen hier im Wesentlichen senkrecht auf dem Abschnitt 30, können zu diesem jedoch auch schräg verlaufen.

Im Querschnitt betrachtet ergibt sich somit im Wesentlichen ein Z-Profil, wobei die im Wesentlichen waagerechten Bereiche des Z durch den zweiten und dritten Abschnitt 32 und 34 und der diese verbindende Mittelsteg durch den ersten Abschnitt 30 gebildet werden.

Ferner weist die Unterschale 26 eine umlaufende Kante 36 auf, die die äußeren Ränder des ersten, zweiten und dritten Abschnitts 30, 32 und 34 bildet. (Fig. 3)

Die in Fig. 4 dargestellte Oberschale 28 weist einen ersten Abschnitt 38 auf, von dessen jeweiligen Enden sich ein zweiter und dritter Abschnitt 40 und 42 quer, d.h. wie hier senkrecht oder auch schräg zu dem ersten Abschnitt 38 in zueinander entgegengesetzten Richtungen weg erstrecken. Aufgrund eines seitlichen Versatzes 44 des ersten Abschnitts 38 in Längserstreckung der Oberschale 28 wird eine Stirnwand 46 ausgebildet.

Die Stirnwand 46 zeigt in distale Richtung und weist eine Austrittsöffnung 48 auf, in der der Lichtleiter 24 angeordnet ist, so dass durch den Lichtleiter 24 hindurchtretendes Licht durch die Austrittsöffnung 48 austreten kann.

Bei einem Einsatz des Laryngoskops 10 kann somit eine Ausleuchtung des Mund- und Rachenraums erreicht werden, wie es bei der Laryngoskopie oder auch bei einer Intubation erforderlich ist.

Der Lichtleiter 24 kann anstelle als Lichtzuführung auch als Bildleiter dienen, wozu in das distale Ende des Lichtleiters 24, das sich in der Austrittsöffnung 48 befindet, Licht in den Lichtleiter 24 eintreten kann. Weiterhin kann es vorgesehen sein, dass in der Austrittsöffnung 48 oder in einer oder mehreren weiteren Austrittsöffnungen eine oder mehrere Lichtquellen, beispielsweise LEDs, oder ein elektronischer Bildaufnehmer angeordnet sind.

Auch die Oberschale 28 weist im Querschnitt im Wesentlichen ein Z-Profil auf, das aus dem zweiten Abschnitt 40, dem ersten Abschnitt 38 und dem dritten Abschnitt 42 gebildet wird.

Die Oberschale 28 weist ferner eine umlaufende Kante 50 auf, die die äußeren Ränder des ersten, zweiten und dritten Abschnitts 38, 40 und 42 bildet.

Wie aus Fig. 2 hervorgeht, liegen die Unterschale 26 und die Oberschale 28 so aneinander, dass sich ein Hohlraum 52 ausbildet.

Dazu ist der dritte Abschnitt 42 der Oberschale 28 an dem dritten Abschnitt 34 der Unterschale 26 sowie der zweite Abschnitt 40 der Oberschale 28 an dem zweiten Abschnitt 32 der Unterschale 26 flächig aneinanderliegend angeordnet. Dabei kommen die umlaufenden Kanten 36 und 50 übereinander zu liegen, so dass sie eine Kontaktkante 54 ausbilden.

Da der dritte Abschnitt 42 der Oberschale 28 kürzer ist als der dritte Abschnitt 34 der Unterschale 26 und der zweite Abschnitt 32 der Unterschale 26 kürzer ist als der zweite Abschnitt 40 der Oberschale 28, sind die ersten Abschnitte 30 und 38 der Unterschale 26 und der Oberschale 28 voneinander beabstandet, wodurch der Hohlraum 52 ausgebildet wird.

Die ersten Abschnitte 30 und 38 bilden somit Seitenwände 56 und 58 des Hohlraums 52.

Durch die Aufeinanderlagerung der zweiten Abschnitte 32 und 40 sowie der dritten Abschnitte 34 und 42 entstehen Schenkel 60 und 62 des Spatelblatts 22. Diese Schenkel 60 und 62 bilden dabei zusammen mit den Seitenwänden 56 und 58 die Begrenzung des Hohlraums 52.

Im Hohlraum 52 verläuft der Lichtleiter 24 ausgehend von z.B. einer Lichtquelle im Griff 12 bis zur bereits erwähnten Austrittsöffnung 48. In den oben genannten Fällen, in denen anstelle des Lichtleiters 24 und zusätzlich zu diesem im distalen Bereich des Spatelblatts 22 eine oder mehrere Lichtquellen, beispielsweise LEDs, oder ein elektronischer Bildaufnehmer angeordnet sind, verlaufen durch den Hohlraum 52 eine oder mehrere elektrische Leitungen, die der Spannungsversorgung der Lichtquelle(n), des elektronischen Bildaufnehmers und als Signalleitungen für den elektrischen Bildaufnehmer dienen.

Die Oberschale 28 und Unterschale 26 sind in dem hier gezeigten Ausführungsbeispiel an der Kontaktkante 54 miteinander durch Laserschweißen verbunden. Dadurch weist das Spatelblatt 22 eine sehr glatte, gleichmäßige Kontaktkante 54 auf, die umlaufend dicht ist und gut zu reinigen ist und an der sich keine Bakterien festsetzen können.

Alternativ kann die Verbindung der Oberschale 28 und der Unterschale 26 auch durch ein Verkleben der aufeinanderliegenden Flächen der zweiten Abschnitte 32 und 40 sowie der dritten Abschnitte 34 und 42 jeweils miteinander oder durch ein Verlöten an der Kontaktkante 54 erfolgen. Die letztere Alternative bedarf danach ggf. einer kurzen Nachbearbeitung zur Glättung der Fügestellen.

Die Unterschale 26 (Fig. 3) und die Oberschale 28 (Fig. 4) sind in diesem Ausführungsbeispiel jeweils aus einem Werkstück durch Hydroforming hergestellt worden. Daher bilden bei der Unterschale 26 die zweiten und dritten Abschnitte 32 und 34 und der erste Abschnitt 30 sowie bei der Oberschale 28 die zweiten und dritten Abschnitte 40 und 42 und der erste Abschnitt 38 mit der Stirnwand 46 jeweils eine durchgehende geschlossene Oberfläche.

Diese Oberfläche ist aufgrund des Hydroformings jeweils glatt und frei von Unebenheiten, die bei anderen Herstellungsverfahren der Schalen auftreten können.

Die Austrittsöffnung 48 in der Stirnwand 46 wird bei Anwendung dieses Verfahrens nachträglich z.B. durch eine Bohrung, durch Stanzen oder durch Laserschneiden eingeführt.

Die hier nicht gezeigte proximale Seite des Hohlraum 52 kann z.B. durch eine nachträglich aufgesetzte Platte oder z.B. auch durch eine ähnlich zur Stirnwand 46 einstückig angeformte Fläche in der Oberschale 28 oder der Unterschale 26 verschlossen sein. Durch Schließen der Verbindungsstelle zwischen dieser Platte bzw. Fläche und der Oberschale 28 und/oder Unterschale 26 durch beispielsweise Laserschweißen wird somit ein vollständig abgedichteter Hohlraum 52 erzeugt, der es gestattet, den Laryngoskopspatel 20 auch mit Dampf zu reinigen. In die proximale Wand des Hohlraums kann eine Öffnung (nicht dargestellt) eingebracht sein, durch die der Lichtleiter 24 und/oder die oben erwähnten elektrischen Leitungen in den Kopf 14 und in den Handgriff 12 verlaufen.

Am distalen Ende weist das Spatelblatt 22 im hier gezeigten Ausführungsbeispiel eine abgerundete Spatelspitze 66 auf (Fig. 1 und Fig. 2). Diese ist hierbei durch Schweißen oder aber auch durch Löten oder Kleben angebracht.

In dem Verfahren zur Herstellung des Spatelblattes 22 des Laryngoskopspatels 20 werden zunächst die Oberschale 28 und die Unterschale 26 durch Hydroforming jeweils aus einem entsprechenden Rohblech geformt.

Hierbei werden der erste Abschnitt 30, der zweite Abschnitt 32 und der dritte Abschnitt 34 der Unterschale 26 geformt. Ebenso werden der erste Abschnitt 38, der zweite Abschnitt 40, der dritte Abschnitt 42 sowie die Stirnwand 46 der Oberschale 28 geformt.

Die so geformten zweiten Abschnitte 32 und 40 und die dritten Abschnitte 34 und 42 werden anschließend flächig aufeinandergelegt, wobei zuvor der Lichtleiter 24 an der ebenfalls zuvor gebohrten Austrittsöffnung 48 befestigt und in dem entstehenden Hohlraum 52 angeordnet wird.

An der durch die Kante 36 der Unterschale 26 und der Kante 50 der Oberschale 28 gebildeten Kontaktkante 54 wird die Unterschale 26 mit der Oberschale 28 durch Laserschweißen miteinander verschweißt.

Am distalen Ende des Spatelblattes 22 wird dann die abgerundete Spatelspitze 66 aufgesetzt und durch anschließendes verschweißen mit dem Spatelblatt 22 verbunden.

Ein ggf. kurzes Nachbearbeiten dieser Schweißnähte erzeugt auch an dieser Stelle glatte gut zu reinigende Übergänge zwischen Spatelblatt 22 und Spatelspitze 66.

Alternativ wird die Spatelspitze 66 bereits im Umformungsschritt, d.h. bei der Herstellung der Oberschale 28 und der Unterschale 26 durch Hydroforming im distalen Ende der Schalen 26 und 28 mit geformt. Dabei kann sie entweder in der Form einer der beiden Schalen 26 oder 28 vorhanden sein oder zu jeweiligen Teilen in beiden Schalen 26 und 28 geformt werden. Ein anschließendes umlaufendes Laserschweißen liefert dann, wie in den zuvor beschriebenen Fällen, ein Spatelblatt, welches glatte Übergänge und keine Unebenheiten aufweist, somit keiner weiteren Nachbearbeitung bedarf und gut zu reinigen ist.

Auf diese Weise wird der auf den Oberflächen aufgrund des Hydroformings und an der Kontaktkante 54 aufgrund des Laserschweißens komplett glatte und gut zu reinigende Laryngoskopspatel 20 hergestellt.

## Patentansprüche

1. Laryngoskopspatel, mit einem Spatelblatt (22), wobei das Spatelblatt (22) im Querschnitt zwei Seitenwände (56, 58), die zumindest über eine Teillänge des Spatelblattes (22) voneinander beabstandet sind, und zwei Schenkel (60, 62) aufweist, von denen ein erster Schenkel (60) sich von der ersten Seitenwand (58) aus über die zweite Seitenwand (56) hinaus erstreckt und ein zweiter Schenkel (62) sich von der zweiten Seitenwand (56) aus über die erste Seitenwand (58) hinaus erstreckt, wobei die Seitenwände (56, 58) und die Schenkel (60, 62) einen Hohlraum (52) ausbilden, **dadurch gekennzeichnet, dass** das Spatelblatt (22) aus einer Ober- (28) und einer Unterschale (26) gebildet ist, die jeweils im Querschnitt einen ersten Abschnitt (30, 38) und zwei zueinander entgegengesetzt verlaufende zweite und dritte Abschnitte (32, 34, 40, 42) aufweisen, wobei die zweiten Abschnitte (32, 40) der Ober- (28) und Unterschale (26) flächig aufeinanderliegend den ersten Schenkel (60) und die dritten Abschnitte (34, 42) flächig aufeinanderliegend den zweiten Schenkel (62) bilden, während die ersten Abschnitte (30, 38) die Seitenwände (56, 58) bilden.

2. Laryngoskopspatel nach Anspruch 1, **dadurch gekennzeichnet, dass** der Hohlraum (52) am distalen Ende durch eine sich quer zur Längsrichtung des Spatelblattes (22) erstreckende Stirnwand (46) abgeschlossen ist, und dass die Stirnwand (46) einstückig mit dem ersten Abschnitt (38) und dem zweiten Abschnitt (40) der Oberschale (28) ausgebildet ist.

3. Laryngoskopspatel nach Anspruch 1 oder 2, **dadurch gekennzeichnet, dass** die Unterschale (26) durch ein Umformverfahren hergestellt ist, wobei der erste Abschnitt (30) mit dem zweiten und dritten Abschnitt (32, 34) einstückig geformt ist.

4. Laryngoskopspatel nach Anspruch 2 oder 3, **dadurch gekennzeichnet, dass** die Oberschale (28) durch ein Umformverfahren hergestellt ist, wobei der erste Abschnitt (38) mit dem zweiten und dritten Abschnitt (40, 42) und der Stirnwand (46) einstückig geformt ist.

5. Laryngoskopspatel nach Anspruch 3 oder 4, **dadurch gekennzeichnet, dass** die Unterschale (26) und/oder die Oberschale (28) durch Hydroforming hergestellt ist.

6. Laryngoskopspatel nach einem der Ansprüche 1 bis 5, **dadurch gekennzeichnet, dass** die Ober- (28) und die Unterschale (26) über die aufeinanderliegenden zweiten und dritten Abschnitte (32, 34, 40, 42) miteinander verklebt sind.

7. Laryngoskopspatel nach einem der Ansprüche 1 bis 5, **dadurch gekennzeichnet, dass** die Ober- (28) und die Unterschale (26) miteinander an einer Kontaktkante (54), die jeweils aneinandergrenzende Kanten (36, 50) der zweiten und dritten Abschnitte (32, 34, 40, 42) miteinander bilden, umlaufend verlötet sind.

8. Laryngoskopspatel nach einem der Ansprüche 1 bis 5, **dadurch gekennzeichnet, dass** die Ober- (28) und die Unterschale (26) miteinander an einer Kontaktkante (54), die jeweils aneinandergrenzende Kanten (36, 50) der zweiten und dritten Abschnitte (32, 34, 40, 42) miteinander bilden, vorzugsweise durch Laserschweißen, umlaufend verschweißt sind.

9. Laryngoskopspatel nach einem der Ansprüche 1 bis 8, **dadurch gekennzeichnet, dass** das Spatelblatt (22) am distalen Ende eine abgerundete Spatelspitze (66) aufweist, und dass die Spatelspitze (66) einstückig mit der Oberschale (28) und/oder der Unterschale (26) geformt ist.

10. Verfahren zum Herstellen eines Spatelblattes (22) eines Laryngoskopspatels, das folgende Schritte aufweist:
- Bereitstellen einer Ober- (28) und einer Unterschale (26), die jeweils im Querschnitt einen ersten Abschnitt (30, 38) und zwei zueinander entgegengesetzt verlaufende zweite und dritte Abschnitte (32, 34, 40, 42) aufweisen,
- flächiges Aufeinanderlegen der zweiten Abschnitte (32, 40) und der dritten Abschnitte (34, 42) von Ober- (28) und Unterschale (26),
- festes Verbinden der Ober- (28) und Unterschale (26) miteinander durch Fügen der zweiten und dritten Abschnitte (32, 34, 40, 42) der Ober- (28) und Unterschale (26), so dass die zweiten Abschnitte (32, 40) einen ersten Schenkel (60) und die dritten Abschnitte (34, 42) einen zweiten Schenkel (62) bilden, und die ersten Abschnitte (30, 38) Seitenwände (56, 58) bilden, wodurch zumindest über eine Teillänge des Spatelblattes ein Hohlraum (52) ausgebildet wird.

11. Verfahren nach Anspruch 10, **dadurch gekennzeichnet, dass** das distale Ende des Hohlraums (52) durch eine sich quer zur Längsrichtung des Spatelblattes (22) erstreckende Stirnwand (46) abgeschlossen wird, die einstückig mit dem ersten Abschnitt (38) und dem zweiten Abschnitt (40) der Oberschale (28) hergestellt wird.

12. Verfahren nach Anspruch 10 oder 11, **dadurch gekennzeichnet, dass** die Unterschale (26) durch ein Umformverfahren hergestellt wird, wobei der erste Abschnitt (30) mit dem zweiten und dritten Abschnitt (32, 34) einstückig geformt wird.

13. Verfahren nach Anspruch 11 oder 12, **dadurch gekennzeichnet, dass** die Oberschale (28) durch ein Umformverfahren hergestellt wird, wobei der erste Abschnitt (38) mit dem zweiten und dritten Abschnitt (40, 42) und der Stirnwand (46) einstückig geformt wird.

14. Verfahren nach Anspruch 12 oder 13, **dadurch gekennzeichnet, dass** die Unterschale (26) und/oder die Oberschale (28) durch Hydroforming hergestellt wird.

15. Verfahren nach einem der Ansprüche 10 bis 14, **dadurch gekennzeichnet, dass** die Ober- (28) und die Unterschale (26) miteinander an einer Kontaktkante (54), die jeweils aneinandergrenzende Kanten (36, 50) der zweiten und dritten Abschnitte (32, 34, 40, 42) miteinander bilden, vorzugsweise durch Laserschweißen, umlaufend verschweißt werden.

16. Verfahren nach einem der Ansprüche 10 bis 15, **dadurch gekennzeichnet, dass** eine am distalen Ende des Spatelblattes (22) angeordnete abgerundete Spatelspitze (66) einstückig mit der Oberschale (28) und/oder der Unterschale (26) geformt wird.

## Claims

1. A laryngoscope spatula, comprising a spatula blade (22), which spatula blade (22), in cross section, has two side walls (56, 58), which are spaced apart from each other along at least part of the length of the spatula blade (22), and two limbs (60, 62), of which a first limb (60) extends from the first side wall (58) to beyond the second side wall (56), and a second limb (62) extends from the second side wall (56) to beyond the first side wall (58), the side walls (56, 58) and the limbs (60, 62) forming a cavity (52), **characterized in that** the spatula blade (22) is composed of an upper shell (28) and a lower shell (26) which each, in cross section, have a first portion (30, 38) and two mutually oppositely directed second and third portions (32, 34, 40, 42), of which the second portions (32, 40) of the upper shell (28) and lower shell (26), lying flat on each other, form the first limb (60), and the third portions (34, 42), lying flat on each other, form the second limb (62), while the first portions (30, 38) form the side walls (56, 58).

2. The laryngoscope spatula of Claim 1, **characterized in that** the cavity (52) is closed off at the distal end by an end wall (46) that extends transversely with respect to the longitudinal direction of the spatula blade (22), and **in that** the end wall (46) is designed in one piece with the first portion (38) and the second portion (40) of the upper shell (28).

3. The laryngoscope spatula of Claim 1 or 2, **characterized in that** the lower shell (26) is produced by a forming method, in which the first portion (30) is formed in one piece with the second and third portions (32, 34).

4. The laryngoscope spatula of Claim 2 or 3, **characterized in that** the upper shell (28) is produced by a forming method, in which the first portion (38) is formed in one piece with the second and third portions (40, 42) and with the end wall (46).

5. The laryngoscope spatula of Claim 3 or 4, **characterized in that** the lower shell (26) and/or the upper shell (28) is produced by hydroforming.

6. The laryngoscope spatula of any one of Claims 1 through 5, **characterized in that** the upper shell (28) and the lower shell (26) are glued to each other via the superposed second and third portions (32, 34,40, 42).

7. The laryngoscope spatula of any one of Claims 1 through 5, **characterized in that** the upper shell (28) and the lower shell (26) are soldered onto each other along a peripheral contact edge (54) formed by mutually adjoining edges (36. 50) of the second and third portions (32, 34, 40, 42).

8. The laryngoscope spatula of any of Claims 1 through 5, **characterized in that** the upper shell (28) and the lower shell (26) are welded onto each other, preferably by laser welding, along a peripheral contact edge (54) formed by mutually adjoining edges (36, 50) of the second and third portions (32, 34, 40, 42).

9. The laryngoscope spatula of any one of Claims 1 through 8, **characterized in that** the spatula blade (22) has a rounded spatula tip (66) at the distal end, and that the spatula tip (66) is formed in one piece with the upper shell (28) and/or the lower shell (26).

10. A method for producing a spatula blade (22) of a laryngoscope spatula, comprising the following steps:
- providing an upper shell (28) and a lower shell (26) which, in cross section, each have a first portion (30, 38) and two mutually oppositely directed second and third portions (32, 34, 40,42),
- laying the second portions (32, 40) and third portions (34, 42) of the upper shell (28) and the lower shell (26) flat on each other,
- firmly connecting the upper shell (28) and lower shell (26) to each other by joining the second and third portions (32, 34, 40, 42) of the upper shell (28) and lower shell (26) such that the second portions (32, 40) form a first limb (60) and the third portions (34, 42) form a second limb (62), and the first portions (30, 38) form side walls (56, 58), as a result of which a cavity (52) is formed along at least part of the length of the spatula blade.

11. The method of Claim 10, **characterized in that** the distal end of the cavity (52) is closed off by an end wall (46) which extends transversely with respect to the longitudinal direction of the spatula blade (22) and which is produced in one piece with the first portion (38) and the second portion (40) of the upper shell (28).

12. The method of Claim 10 or 11, **characterized in that** the lower shell (26) is produced by a forming method, in which the first portion (30) is formed in one piece with the second and third portions (32, 34).

13. The method of Claim 11 or 12, **characterized in that** the upper shell (28) is produced by a forming method, in which the first portion (38) is formed in one piece with the second and third portions (40, 42) and with the end wall (46).

14. The method of Claim 12 or 13, **characterized in that** the lower shell (26) and/or the upper shell (28) is produced by hydroforming.

15. The method of any one of Claims 10 through 14, **characterized in that** the upper shell (28) and the lower shell (26) are welded onto each other, preferably by laser welding, along a peripheral contact edge (54) formed by mutually adjoining edges (36, 50) of the second and third portions (32, 34, 40, 42).

16. The method of any one of Claims 10 through 15, **characterized in that** a rounded spatula tip (66) arranged at the distal end of the spatula blade (22) is formed in one piece with the upper shell (28) and/or the lower shell (26).

## Revendications

1. Spatule de laryngoscope, avec une lame de spatule (22), la lame de spatule (22) comportant deux parois latérales (56, 58), qui sont distantes l'une de l'autre au moins sur une longueur partielle de la lame de spatule (22), et deux branches (60, 62), parmi lesquelles une première branche (60) s'étend depuis la première paroi latérale (58) au-delà de la deuxième paroi latérale (56) et une deuxième branche (62) s'étend depuis la deuxième paroi latérale (56) au-delà de la première paroi latérale (58), les parois latérales (56, 58) et les branches (60, 62) formant une cavité (52), **caractérisée en ce que** la lame de spatule (22) est formée d'une coque supérieure (28) et d'une coque inférieure (26) qui comportent chacune, en coupe transversale, une première partie (30, 38) et des deuxième et troisième parties (32, 34, 40, 42) s'étendant en opposition mutuelle, les deuxièmes parties (32, 40) de la coque supérieure (28) et de la coque inférieure (26) formant en reposant à plat l'une sur l'autre la première branche (60) et les troisièmes parties (34, 42) formant en reposant à plat l'une sur l'autre la deuxième branche (62), tandis que les premières parties (30, 38) forment les parois latérales (56, 58).

2. Spatule de laryngoscope selon la revendication 1, **caractérisée en ce que** la cavité (52) est fermée à l'extrémité distale par une paroi frontale (46) s'étendant transversalement à la direction longitudinale de la lame de spatule (22), et **en ce que** la paroi frontale (46) est réalisée d'un seul tenant avec la première partie (38) et la deuxième partie (40) de la coque supérieure (28).

3. Spatule de laryngoscope selon la revendication 1 ou 2, **caractérisée en ce que** la coque inférieure (26) est fabriquée par un procédé de formage, la première partie (30) étant formée d'un seul tenant avec la deuxième et la troisième parties (32, 34).

4. Spatule de laryngoscope selon la revendication 2 ou 3, **caractérisée en ce que** la coque supérieure (28) est fabriquée par un procédé de formage, la première partie (38) étant formée d'un seul tenant avec la deuxième et la troisième parties (40, 42) et avec la paroi frontale (46).

5. Spatule de laryngoscope selon la revendication 3 ou 4, **caractérisée en ce que** la coque inférieure (26) et/ou la coque supérieure (28) sont fabriquées par formage hydraulique.

6. Spatule de laryngoscope selon l'une quelconque des revendications 1 à 5, **caractérisée en ce que** la coque supérieure (28) et la coque inférieure (26) sont assemblées entre elles par collage par l'intermédiaire des deuxièmes et troisièmes parties (32, 34, 40, 42) reposant à plat les unes sur les autres.

7. Spatule de laryngoscope selon l'une quelconque des revendications 1 à 5, **caractérisée en ce que** la coque supérieure (28) et la coque inférieure (26) sont assemblées entre elles par brasage périphérique au niveau d'un bord de contact (54) que forment ensemble des bords respectivement adjacents (36, 50) des deuxièmes et troisièmes parties (32, 34, 40, 42).

8. Spatule de laryngoscope selon l'une quelconque des revendications 1 à 5, **caractérisée en ce que** la coque supérieure (28) et la coque inférieure (26) sont assemblées entre elles par soudage périphérique, de préférence par soudage au laser, au niveau d'un bord de contact (54) que forment ensemble des bords respectivement adjacents (36, 50) des deuxièmes et troisièmes parties (32, 34, 40, 42).

9. Spatule de laryngoscope selon l'une quelconque des revendications 1 à 8, **caractérisée en ce que** la lame de spatule (22) comporte à l'extrémité distale une pointe de spatule (66) arrondie, et **en ce que** la pointe de spatule (66) est formée d'un seul tenant avec la coque supérieure (28) et/ou la coque inférieure (26).

10. Procédé de fabrication d'une lame de spatule (22) d'une spatule de laryngoscope, comprenant les étapes suivantes :
- mise à disposition d'une coque supérieure (28) et d'une coque inférieure (26) qui comportent chacune, en coupe transversale, une première partie (30, 38) et des deuxième et troisième parties (32, 34, 40, 42) s'étendant en opposition mutuelle,
- application à plat l'une sur l'autre des deuxièmes parties (32, 40) et des troisièmes parties (34, 42) de la coque supérieure (28) et de la coque inférieure (26),
- assemblage mutuel fixe de la coque supérieure (28) et de la coque inférieure (26) en joignant les deuxièmes et troisièmes parties (32, 34, 40, 42) de la coque supérieure (28) et de la coque inférieure (26), de sorte que les deuxièmes parties (32, 40) forment une première branche (60) et les troisièmes parties (34, 42) une deuxième branche (62), et que les premières parties (30, 38) forment des parois latérales (56, 58), une cavité (52) étant ainsi formée au moins sur une longueur partielle de la lame de spatule.

11. Procédé selon la revendication 10, **caractérisé en ce que** l'extrémité distale de la cavité (52) est fermée par une paroi frontale (46) s'étendant transversalement à la direction longitudinale de la lame de spatule (22) et réalisée d'un seul tenant avec la première partie (38) et la deuxième partie (40) de la coque supérieure (28).

12. Procédé selon la revendication 10 ou 11, **caractérisé en ce que** la coque inférieure (26) est fabriquée par un procédé de formage, la première partie (30) étant formée d'un seul tenant avec la deuxième et la troisième parties (32, 34).

13. Procédé selon la revendication 11 ou 12, **caractérisé en ce que** la coque supérieure (28) est fabriquée par un procédé de formage, la première partie (38) étant formée d'un seul tenant avec la deuxième et la troisième parties (40, 42) et avec la paroi frontale (46).

14. Procédé selon la revendication 12 ou 13, **caractérisé en ce que** la coque inférieure (26) et/ou la coque supérieure (28) sont fabriquées par formage hydraulique.

15. Procédé selon l'une quelconque des revendications 10 à 14, **caractérisé en ce que** la coque supérieure (28) et la coque inférieure (26) sont assemblées entre elles par soudage périphérique, de préférence par soudage au laser, au niveau d'un bord de contact (54) que forment ensemble des bords respectivement adjacents (36, 50) des deuxièmes et troisièmes parties (32, 34, 40, 42).

16. Procédé selon l'une quelconque des revendications 10 à 15, **caractérisé en ce qu'**une pointe de spatule (66) arrondie disposée à l'extrémité distale de la lame de spatule (22) est formée d'un seul tenant avec la coque supérieure (28) et/ou la coque inférieure (26).
